# EUROPEAN PATENT SPECIFICATION

(11) **EP 3 159 277 B1**
(45) Date of publication and mention of the grant of the patent: **29.05.2019**
(21) Application number: 16194967.2
(22) Date of filing: 21.10.2016
(51) Int. Cl.: B65D 75/32

(54) **BLISTER FOR INHALABLE FORMULATION OF TIOTROPIUM BROMIDE**
BLISTER FÜR INHALIERBARE FORMULIERUNG VON TIOTROPIUMBROMID
BLISTER POUR FORMULATION INHALABLE DE BROMURE DE TIOTROPIUM

(30) Priority: 23.10.2015 TR 201513285
(43) Date of publication of application: 26.04.2017
(73) Proprietor: Arven Ilac Sanayi Ve Ticaret A.S., Istanbul 34460 (TR)
(72) Inventor: TÜRKYILMAZ, Ali, 34460 Istanbul (TR); CELIK, Devrim, 34460 Istanbul (TR)
(74) Representative: Sevinç, Erkan

(56) References cited:
- WO-A1-03/078429
- WO-A1-2013/109219
- US-A1- 2014 197 066

## Description

### Field of Invention

This invention is about a blister which is an integral component of a dry powder inhaler, structured from a base sheet and a lid sheet comprising at least two layers made from polymeric materials and formed or flat aluminium foil.

### Background of Invention

Asthma and chronic obstructive pulmonary disease (COPD) affect more than 30 million people in the United States. More than 100,000 deaths each year are attributable to these conditions. Obstruction to airflow through the lungs is the characteristic feature in each of these airway diseases, and the medications utilized in treatment are often similar. Chronic obstructive pulmonary disease (COPD) is a widespread chronic lung disorder encompassing chronic bronchitis and emphysema. The causes of COPD are not fully understood. Experience shows that the most important cause of chronic bronchitis and emphysema is cigarette smoking. Air pollution and occupational exposures may also play a role, especially when combined with cigarette smoking. Heredity also causes some emphysema cases, due to alpha1 anti-trypsin deficiency.

Administration of asthma drugs by an oral inhalation route is very much in focus today, because of advantages offered like rapid and predictable onset of action, cost effectiveness and high level of comfort for the user. Dry powder inhalers (DPI) are especially interesting as an administration tool, compared to other inhalers, because of the flexibility they offer in terms of nominal dose range, i.e. the amount of active substance that can be administered in a single inhalation.

Dry powder inhalers (DPI) are becoming more and more popular because of their ease of use and medical efficacy. DPI's can be divided into two major categories: bulk and pre-metered devices. Pre-metered devices are gaining more and more market share due to the ability to control the product and process of metering a correct dose to the user. DPIs with pre-metered doses are, because of this, more reliable than bulk inhalers that meter the powder dose inside the inhaler. A pre-metered DPI moves the critical step of metering a dose to a pharmaceutical production process.

Anticholinergic agents, e.g. tiotropium, especially tiotropium bromide, are effective bronchodilators. These medicaments have a relatively fast onset and long duration of action, especially tiotropium bromide, which may be active for up to 24 hours. Anticholinergic agents reduce vagal cholinergic tone of the smooth muscle, which is the main reversible component of COPD. Anticholinergic agents have been shown to cause quite insignificant side effects in clinical testing, dryness of mouth and constipation are perhaps the most common symptoms. Because it is often very difficult to diagnose asthma and COPD correctly and since both disorders may co-exist, it is advantageous to treat patients suffering temporary or continuous bronchial obstruction resulting in dyspnoea with a small but efficient dose of a long-acting anticholinergic agent, preferably tiotropium bromide, because of the small adverse side effects.

Tiotropium bromide is known from European Patent Application EP 418 716 A1 and has the following chemical structure shown as Formula.I. Tiotropium bromide is the preferred anticholinergic agent because of its high potency and long duration. However, tiotropium is difficult to formulate in dry powder form to provide acceptable performance in terms of dose efficacy using prior art DPIs. Dose efficacy depends to a great deal on delivering a stable and high fine particle dose (FPD) out of the dry powder inhaler. It is also important to keep the dosage to the user as exact as possible and to maintain a stable efficacy over time, and that the medicament dose does not deteriorate during normal storage.

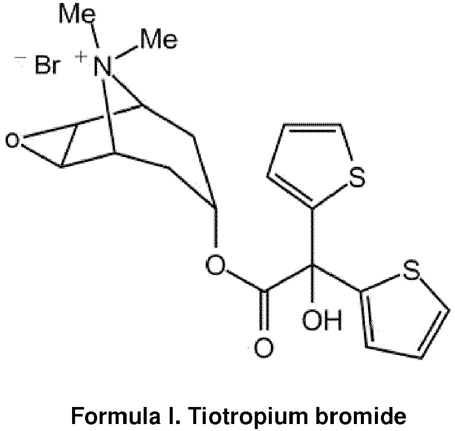

Tiotropium in pure form is a potent drug and it is therefore diluted before a dose forming step by mixing with acceptable excipients, in selected ratio (s) in order to fit a preferred method of dose forming and loading. For example, details about inhalation powders containing tiotropium in mixtures with excipients, methods of powder manufacture, use of powder and capsules for powder may be studied in the international publication WO 02/30389 A1, Bechtold-Peters et al. Manufacturing a formulation of a very small amount of e. g. tiotropium with a much larger quantity of excipient requires special precautions to be taken to give a final, stable and robust manufacturing method.

A prior art blister is disclosed in WO 2013/109219 A1. This document relates to pharmaceutical formulations in dry powder form comprising tiotropium and carmoterol and/or pharmaceutically acceptable derivatives thereof in order to be used in symptomatic and/or prophylactic treatment of respiratory tract diseases, particularly asthma and COPD. Dry powder formulations comprising tiotropium, carmoterol, fine grained lactose and coarse grained lactose are disclosed within WO 2013/109219 A1. The main problem addressed in WO 2013/109219 A1 is mentioned as poor flow characteristics of dry powder formulations. Specific weight ratio between tiotropium and carmoterol and specific particle size ratio of fine grained lactose to coarse grained lactose are proposed to in order to solve flowability problems.

Excipients are particularly necessary in powders for inhalation if the efficacy of the pharmaceutical substance used is very high like tiotropium, so that only small amounts are needed for each individual dose. Preparing a formulation of tiotropium and an excipient where the amount of tiotropium is very small (e.g., < 1: 100 the amount of the excipient). In this case it is advisable to dilute the active substance so as to achieve good accuracy of flowability and metering. Additionally, maintaining the stability and preventing the agglomeration of dry powder formulation which is contained in a blister strip that is prepared for use in a single dose dry powder inhaler in order to achieve an effective inhalation should be considered.

One of the disadvantages of that method is that large amounts of excipient must be added to the formulation, to achieve the desired flowability. Because of the large amount of excipient, the properties of the inhalable powder are critically influenced by the choice of excipient. Further, the correct metered dose loaded into an inhaler to be used for the purpose of administration must be adjusted for predicted losses such as retention and more or less efficient de-aggregation of the inhaled dose.

Tiotropium is known in the industry to have problems maintaining in-use stability due to sensitivity to moisture. Conventional packaging into gelatin capsules or blister show poor performance and have a short in-use stability.

There is still need for improvement to dilute potent tiotropium and to make flowability of dry powder formulation acceptable for the dose forming process and optimize the delivered dose and maintain stable efficacy.

### Description of the invention

This invention is about a blister which is an integral component of a dry powder inhaler, structured from a base sheet and a lid sheet comprising at least two layers made from polymeric materials and formed or flat aluminium foil, in accordance with appended claim 1.

In a preferred embodiment, the cavity is preferably in the volume range of 10 to 19.8 mm³, more preferably in the range of 15 to 19.7 mm³.

In this present invention, blister cavity is decreased and cavity filling percentage is increased. As a result of this, there is less space in every blister cavity and tiotropium exposes to air less. The inhalable powder is protected from moisture in this smaller cavities, so stability is improved.

According to this embodiment, the blister is in a strip form. And it comprises two sheets. The lid sheet and the base sheet of the blister strip, consist of at least two layers such as polymeric layer, aluminium foil.

According to the present invention, the polymeric materials used for making the polymeric layers which are present in both the base sheet and lid sheet are selected from the group consisting of polyethylene, polypropylene, polystyrene, polyolefin, polyamide, polyvinyl chloride, polyurethane, vinyl acetate, polybutylmethacrylate, copolymer of vinyl chloride, polyethylene terephthalate (PET), polyvinyl chloride (PVC), ortho-phthalic aldehyde (OPA) or mixtures thereof.

According to the invention, aluminium foil is used both in the lid sheet and in the base sheet of the blister strip to provide high humidity and gas protection. These layers must have the sufficient thickness which provides the protection for the stability of humidity sensitive dry powder formulation which is stored in blister cavity.

According to the embodiment, aluminium foil that is used in the lid sheet of the blister strip is in the thickness range of 5 to 40 µm, preferably of 10 to 30 µm.

In another embodiment, aluminium foil that is used in the base sheet of the blister strip is in the thickness range of 30 to 60 µm, preferably of 35 to 55 µm.

The polymeric layers which are contained in the lid sheet and the base sheet of the blister strip in accordance with the present invention may be made from either same of different polymers. The thickness of these polymeric layers depends on the type of polymeric substance used and its properties.

According to the embodiment, the polymeric layer which is used in the lid sheet of the blister strip is in the thickness range of 10 to 50 µm, preferably of 15 to 35 µm depending on the type of polymer used. The polymeric material used for making the polymeric layer is preferably polyethylene terephthalate (PET).

In another embodiment, the polymeric layer which is used in the base sheet of the blister strip is in the thickness range of 10 to 40 µm, preferably of 15 to 35 µm depending on the type of polymer used. The polymeric material used for making the polymeric layer is preferably ortho-phthalic aldehyde (OPA).

In one embodiment, the polymeric layer which is used in the base sheet of the blister strip is in the thickness range of 80 to 120 µm, preferably of 90 to 110 µm depending on the type of polymer used. The polymeric material used for making the polymeric layer is preferably polyvinyl chloride (PVC).

Moreover, blisters which placed in the blister strips, can be in any shape wherein the polymeric layer forms a molded cavity.

The inside layer of blister cavity of said blister strip which is in contact with dry powder formulation is polymeric layer because of the fact that aluminium foil causes adhesion of part of dry powder formulation to inside layer of the cavity due to electrostatic forces, and hence cause uncontrolled dosing.

In one aspect, the present description provides the dry powder formulation which contain: tiotropium and is inhaled from the blisters. In another aspect, the present invention provides the dry powder formulation which contains tiotropium and inhaled by providing controlled dosing of the dry powder formulation in an effective and stable way.

In another aspect, the present description provides the dry powder formulation which contains tiotropium and is inhaled in an effective, hygienic, user-friendly way.

A blister for use in a dry powder inhaler contains an inhalable formulation comprising tiotropium or an acceptable salt, solvate, hydrate thereof and at least one pharmaceutically acceptable excipient.

The inhalable formulation comprises tiotropium or an acceptable salt, solvate, hydrate is tiotropium bromide.

In a preferred embodiment, the blister contains an inhalable formulation comprising tiotropium bromide and at least one pharmaceutically acceptable excipient. According to the invention, said dry powder formulation comprising tiotropium bromide in an amount in the range of 0.02 to 0.03 mg.

According to this embodiment, the amount of tiotropium bromide is present in an amount of 0.25 to 0.75 % by weight of total composition; preferably it is 0.35 to 0.45% by weight of total composition.

With active substances which have a particularly high efficacy, only small amounts of the active substance are needed per single dose to achieve the desired therapeutic effect. In such cases, the active substance has to be diluted with suitable excipients in order to prepare the inhalable powder. Because of the large amount of excipient, the properties of the inhalable powder are critically influenced by the choice of excipient. However, good flow properties are a prerequisite for highly accurate metering when packing and dividing up the individual doses of preparation, e. g. when producing capsules for powder inhalation or when the patient is metering the individual dose before using a multi-dose inhaler. The term inhalable proportion of active substance refers to the particles of the inhalable powder which are conveyed deep into the branches of the lungs when inhaled with a breath.

The inhalable formulation comprising tiotropium includes at least one pharmaceutically acceptable excipient is selected from the group consisting of monosaccarides, disaccarides, polylactides, oligo-and polysaccarides, polyalcohols, glucose, arabinose, lactose, lactose monohydrate, lactose anhydrous, saccharose, maltose, dextrane, sorbitol, mannitol, xylitol, sodium chloride, calcium carbonate or mixtures thereof.

A particular excipient is lactose. In our findings regarding the sensitivity to moisture for tiotropium powders the moisture properties of any proposed excipient must be appropriate before it is selected for inclusion in a formulation comprising tiotropium, regardless of the function of the proposed excipient. An excipient which, after dose forming, gives off much water inside the container enclosing the dose of mixed powders may negatively affect the included active powder, such that the resulting fine particle dose (FPD) deteriorates rapidly after dose forming.

Therefore, excipients to be mixed with tiotropium must be selected primarily among acceptable excipients, which have good moisture qualities in the sense that the substance will not adversely affect the active medicament fine particle dose (FPD) for the shelf life of the product regardless of normal changes in ambient conditions during storage. Suitable "dry" excipients include those in the above-mentioned groups.

In a preferred embodiment at least one pharmaceutically acceptable excipient is lactose monohydrate. Lactose is selected as the dry excipient and the excipient is preferably lactose monohydrate to be used in a mixture with tiotropium. One reason for selecting lactose as excipient is its inherent property of having a low and constant water sorption isotherm.

Excipients having a similar or lower sorption isotherm may also be considered for use, provided other required qualities are met.

Ambient conditions during dose forming, loading and container sealing should be closely controlled. The temperature should preferably be below 25ºC and relative humidity should preferably be below 35 % Rh. The powder formulation should also be kept as dry as possible during the dose forming process. Taking these precautions will ensure that only a very small, acceptable amount of water is enclosed in the container together with the dose and not enough to present a threat to the stability of the moisture sensitive substance and the fine particle dose (FPD). The original fine particle fraction (FPF) of the medicament dose (e. g. tiotropium) manifested in a high fine particle dose (FPD) of the metered dose of the medical product at the packaging stage is preserved in the high barrier seal container. Thus, when the pre-metered dose is delivered by a dry powder inhaler (DPI) it is unaffected for the shelf life of the medical product by normal variations in ambient conditions during handling, storage and delivery.

In order to develop a formulation of tiotropium having controlled moisture properties a study into the chemical and physical properties of the chosen excipient should first be carried out. The sorption isotherm properties will give information with respect to how a formulation will respond to different temperatures and relative humidity in its surrounding environment. One very important question is also the "memory" of some excipients built in by the fact that it takes a very long time to reach steady state for the excipient after a disturbance in the environment. A suitable excipient for a formulation comprising tiotropium is an excipient like lactose monohydrate. The isotherm of lactose monohydrate has three important properties:
- Low absolute water content
- Low change in absolute water content after a change in relative humidity.
- Highly stable in in-use temperature situations

Low absolute water content ensures that a disturbance from steady conditions will not have a big impact on a tiotropium dose when the total amount of water present in the excipient is low. The low change in absolute water content at different relative humidity ensures that the excipient has no "memory" and that it can easily be put into a steady state at a given relative humidity before filling into a high barrier container. The temperature stability ensures that adsorption and desorption inside the high barrier seal will influence the pharmacologically active ingredient (API) as little as possible.

According to this embodiment, the amount of lactose monohydrate is present in an amount of 98.00 to 99.99 % by weight of total composition; preferably it is 99.00 to 99.99% by weight of total composition.

Said dry powder formulation comprises lactose monohydrate in an amount in the range of 2.9 to 5.9 mg, preferably in an amount in the range of 5.0 to 5.29 mg.

In this present invention, smaller amount of excipient is used for dilution and achieved desired flowability. Therefore total amount of the formulation is decreased with small amount of excipient. In addition, smaller amount of excipient provides less side effects.

In this present invention, vacuum-drum technology is used for dose forming process. In this method, composition is vacuumed and filled into separate cavities. Drums are specialized for this inhalable powder characteristics comprising tiotropium.

### Example : Tiotropium bromide dry powder inhaler

| **Ingredients** | **Amount (mg)** |
|---|---|
| tiotropium bromide | 0.0217 |
| lactose monohydrate | 5.2783 |

### Manufacturing Process:

- Lactose monohydrate is weighted into a glass container.
- The walls of shaker container are plastered with a sum of lactose monohydrate.
- Lactose monohydrate and Tiotropium bromide are weighed and added into the shaker container.
- A sum of lactose monohydrate is added into the shaker container and mixed for 3 minutes. This process is repeated two times more with lactose monohydrate again.
- Obtained mixture is sieved through 125 µm hand sieve to obtain a homogenous mixture without causing no particle size change.
- The whole mixture is taken into the same shaker container again.
- The sieve is cleaned with rest amount of lactose monohydrate.
- The mixture is mixed for 105 minutes with shaker container. In order to reduce electrostatic charge of the micronized active substance and to provide mixing homogenously, "electrostatic eliminator" is used during the process.

### Blister Filling Process:

Vacuum-drum technology dose forming process is used for blister filling. The blister cavity is in the volume range of 15 to 19.7 mm3 and the cavity is filled preferably up to 40-60 % by vacuum-drum technology dose forming process. The powder which is filled into the cavity is in the amount of 3-6 mg.

During the process parameters of vacuum-drum device are;
- Vacuum pressure: -50 - 400 mBar
- Blow pressure: 0.2 - 2.0 bar
- In-drum cleaning: 0.5 - 2.0 bar
- Dosing rate: 5-35 period/min
- Sealing temperature: 140-200 °C
- Compressive strength: 10-30 kN
- Sealing time: 200-600 ms

Finally, the blister strips are coiled into the Sanohaler medical device.

## Claims

1. A blister which is an integral component of a dry powder inhaler is structured from a base sheet and a lid sheet comprising at least two layers made from formed polymeric materials or flat aluminium foil, wherein a cavity is filled up to 40-60 % with an inhalable formulation which contains tiotropium bromide in an amount of 0.25 to 0.75 % by weight of the total composition and at least one pharmaceutically acceptable excipient, also said cavity is filled about 3-6 mg of the formulation, and the cavity is in the volume range of 5 to 19.8 mm³.

2. The blister according to claim 1, wherein the cavity is in the volume range of 10 to 19.8 mm³, preferably in the range of 15 to 19.7 mm³.

3. The blister according to claim 1, wherein the blister is in a strip form.

4. The blister according to claim 1, wherein the polymeric materials used for making the polymeric layers which are present in both the base sheet and lid sheet are selected from the group consisting of polyethylene, polypropylene, polystyrene, polyolefin, polyamide, polyvinyl chloride, polyurethane, vinyl acetate, polybutylmethacrylate, copolymer of vinyl chloride, polyethylene terephthalate (PET), polyvinyl chloride (PVC), ortho-phthalic aldehyde (OPA) or mixtures thereof.

5. The blister according to claim 1, wherein aluminium foil that is used in the lid sheet of the blister strip is in the thickness range of 5 to 40 µm, preferably of 10 to 30 µm.

6. The blister according to claim 1, wherein aluminium foil that is used in the base sheet of the blister strip is in the thickness range of 30 to 60 µm, preferably of 35 to 55 µm.

7. The blister according to claim 1, wherein the polymeric layer which is used in the lid sheet of the blister strip is in the thickness range of 10 to 50 µm, preferably of 15 to 35 µm.

8. The blister according to claim 4 and 7, wherein the polymeric material used for making the polymeric layer is polyethylene terephthalate (PET).

9. The blister according to claim 1, wherein the polymeric layer which is used in the base sheet of the blister strip is in the thickness range of 10 to 40 µm, preferably of 15 to 35 µm.

10. The blister according to claim 4 and 9, wherein the polymeric material used for making the polymeric layer is ortho-phthalic aldehyde (OPA).

11. The blister according to claim 1, further comprising another polymeric layer which is used in the base sheet of the blister strip and is in the thickness range of 80 to 120 µm, preferably of 90 to 110 µm.

12. The blister according to claim 4 and 11, wherein the polymeric material used for making the polymeric layer is polyvinyl chloride (PVC).

13. The blister according to claim 1, wherein tiotropium bromide is present in an amount of 0.35 to 0.45% by weight of total composition.

14. The blister according to claim 1, wherein the at least one pharmaceutically acceptable excipient is lactose monohydrate.

15. The blister according to claim 14, wherein lactose monohydrate is present in an amount of 98.00 to 99.99 % by weight of total composition; preferably it is 99.00 to 99.99% by weight of total composition.

## Patentansprüche

1. Ein Blister, der eine integrale Komponente eines Trockenpulver-Inhalators ist, ist aus einem Basisbogen und einem Deckelbogen, umfassend mindestens zwei Schichten, die aus geformten polymeren Materialien oder flacher Aluminiumfolie gebildet sind, aufgebaut, wobei eine Kavität mit bis zu 40 - 60 % einer inhalierbaren Formulierung, welche Tiotropiumbromid in einer Menge von 0,25 bis 0,75 %, bezogen auf das Gewicht der Gesamtzusammensetzung, und mindestens einen pharmazeutisch annehmbaren Hilfsstoff enthält, befüllt ist, wobei diese Kavität auch mit etwa 3-6 mg der Formulierung befüllt ist, und die Kavität in dem Volumenbereich von 5 bis 19,8 mm³ ist.

2. Der Blister gemäß Anspruch 1, wobei die Kavität in dem Volumenbereich von 10 bis 19,8 mm³ ist, bevorzugt in dem Bereich von 15 bis 19,7 mm³.

3. Der Blister gemäß Anspruch 1, wobei der Blister in einer Streifenform ist.

4. Der Blister gemäß Anspruch 1, wobei die polymeren Materialien, die für die Herstellung der polymeren Schichten verwendet werden, welche sowohl in dem Basisbogen als auch dem Deckelbogen vorliegen, ausgewählt sind aus der Gruppe, bestehend aus Polyethylen, Polypropylen, Polystyrol, Polyolefin, Polyamid, Polyvinylchlorid, Polyurethan, Vinylacetat, Polybutylmethacrylat, Copolymer von Vinylchlorid, Polyethylenterephthalat (PET), Polyvinylchlorid (PVC), ortho-Phthalsäurealdehyd (OPA) oder Mischungen davon.

5. Der Blister gemäß Anspruch 1, wobei die Aluminiumfolie, die in dem Deckelbogen des Blisterstreifens verwendet wird, in dem Dickenbereich von 5 bis 40 µm, bevorzugt von 10 bis 30 µm, ist.

6. Der Blister gemäß Anspruch 1, wobei die Aluminiumfolie, die in dem Basisbogen des Blisterstreifens verwendet wird, in dem Dickenbereich von 30 bis 60 µm, bevorzugt von 35 bis 55 µm, ist.

7. Der Blister gemäß Anspruch 1, wobei die polymere Schicht, welche in dem Deckelbogen des Blisterstreifens verwendet wird, in dem Dickenbereich von 10 bis 50 µm, bevorzugt von 15 bis 35 µm, ist.

8. Der Blister gemäß Anspruch 4 und 7, wobei das polymere Material, das für die Herstellung der polymeren Schicht verwendet wird, Polyethylenterephthalat (PET) ist.

9. Der Blister gemäß Anspruch 1, wobei die polymere Schicht, welche in dem Basisbogen des Blisterstreifens verwendet wird, in dem Dickenbereich von 10 bis 40 µm, bevorzugt von 15 bis 35 µm, ist.

10. Der Blister gemäß Anspruch 4 und 9, wobei das polymere Material, das für die Herstellung der polymeren Schicht verwendet wird, ortho-Phthalsäurealdehyd (OPA) ist.

11. Der Blister gemäß Anspruch 1, weiterhin umfassend eine andere polymere Schicht, welche in dem Basisbogen des Blisterstreifens verwendet wird, und in dem Dickenbereich von 80 bis 120 µm, bevorzugt von 90 bis 110 µm, ist.

12. Der Blister gemäß Anspruch 4 und 11, wobei das polymere Material, das für die Herstellung der polymeren Schicht verwendet wird, Polyvinylchlorid (PVC) ist.

13. Der Blister gemäß Anspruch 1, wobei Tiotropiumbromid in einer Menge von 0,35 bis 0,45 %, bezogen auf das Gewicht der Gesamtzusammensetzung, vorhanden ist.

14. Der Blister gemäß Anspruch 1, wobei der mindestens eine pharmazeutisch annehmbare Hilfsstoff Lactosemonohydrat ist.

15. Der Blister gemäß Anspruch 14, wobei Lactosemonohydrat in einer Menge von 98,00 bis 99,99 %, bezogen auf das Gewicht der Gesamtzusammensetzung, vorhanden ist; bevorzugt ist es 99,00 bis 99,99 %, bezogen auf das Gewicht der Gesamtzusammensetzung.

## Revendications

1. Blister qui fait partie intégrante d'un inhalateur de poudre sèche, structuré à partir d'une feuille de base et d'une feuille d'operculage comprenant au moins deux couches faites de matières polymères façonnées ou d'un papier d'aluminium plat, dans lequel une cavité est remplie jusqu'à 40-60 % par une formulation inhalable qui contient du bromure de tiotropium dans une quantité de 0,25 à 0,75 % en poids de la composition totale et au moins un excipient pharmaceutiquement acceptable, également ladite cavité étant remplie d'environ 3-6 mg de la formulation, et la cavité étant dans la plage de volume de 5 à 19,8 mm³.

2. Blister selon la revendication 1, dans lequel la cavité est dans la plage de volume de 10 à 19,8 mm³, de préférence dans la plage de 15 à 19,7 mm³.

3. Blister selon la revendication 1, dans lequel le blister est dans une forme de bande.

4. Blister selon la revendication 1, dans lequel les matières polymères utilisées pour fabriquer les couches polymères qui sont présentes à la fois dans la feuille de base et dans la feuille d'operculage sont choisies dans le groupe consistant en polyéthylène, polypropylène, polystyrène, polyoléfine, polyamide, poly(chlorure de vinyle), polyuréthane, acétate de vinyle, poly(méthacrylate de butyle), copolymère de chlorure de vinyle, poly(téréphtalate d'éthylène) (PET), poly(chlorure de vinyle) (PVC), aldéhyde ortho-phtalique (OPA) ou leurs mélanges.

5. Blister selon la revendication 1, dans lequel le papier d'aluminium qui est utilisée dans la feuille d'operculage de la bande de blister est dans la plage d'épaisseur de 5 à 40 µm, de préférence de 10 à 30 µm.

6. Blister selon la revendication 1, dans lequel le papier d'aluminium qui est utilisée dans la feuille de base de la bande de blister est dans la plage d'épaisseur de 30 à 60 µm, de préférence de 35 à 55 µm.

7. Blister selon la revendication 1, dans lequel la couche polymère qui est utilisée dans la feuille d'operculage de la bande de blister est dans la plage d'épaisseur de 10 à 50 µm, de préférence de 15 à 35 µm.

8. Blister selon l'une des revendications 4 et 7, dans lequel la matière polymère utilisée pour fabriquer la couche polymère est le poly(téréphtalate d'éthylène) (PET).

9. Blister selon la revendication 1, dans lequel la couche polymère qui est utilisée dans la feuille de base de la bande de blister est dans la plage d'épaisseur de 10 à 40 µm, de préférence de 15 à 35 µm.

10. Blister selon l'une des revendications 4 et 9, dans lequel la matière polymère utilisée pour fabriquer la couche polymère est l'aldéhyde ortho-phtalique (OPA).

11. Blister selon la revendication 1, comprenant en outre une autre couche polymère qui est utilisée dans la feuille de base de la bande de blister et est dans la plage d'épaisseur de 80 à 120 µm, de préférence de 90 à 110 µm.

12. Blister selon l'une des revendications 4 et 11, dans lequel la matière polymère utilisée pour fabriquer la couche polymère est le poly(chlorure de vinyle) (PVC).

13. Blister selon la revendication 1, dans lequel le bromure de tiotropium est présent dans une quantité de 0,35 à 0,45 % en poids de la composition totale.

14. Blister selon la revendication 1, dans lequel ledit au moins un excipient pharmaceutiquement acceptable est le lactose monohydraté.

15. Blister selon la revendication 14, dans lequel le lactose monohydraté est présent dans une quantité de 98,00 à 99,99 % en poids de la composition totale, de préférence dans une quantité de 99,00 à 99,99 % en poids de la composition totale.
